Europäisches Patentamt

European Patent Office

Office européen des brevets

(19)

(11) Numéro de publication: **0 265 327 B1**

(12) **FASCICULE DE BREVET EUROPEEN**

(45) Date de publication du fascicule du brevet:
**26.09.90**

(51) Int. Cl.⁵: **G01N 33/78**

(21) Numéro de dépôt: **87402283.3**

(22) Date de dépôt: **13.10.87**

(54) **Procédé de dosage de la parathormone.**

(30) Priorité: **13.10.86 FR 8614184**

(43) Date de publication de la demande:
**27.04.88 Bulletin 88/17**

(45) Mention de la délivrance du brevet:
**26.09.90 Bulletin 90/39**

(84) Etats contractants désignés:
**AT BE CH DE ES FR GB GR IT LI LU NL SE**

(56) Documents cités:
**US-A- 4 369 138**
**US-A- 4 508 828**

**CLINICAL CHEMISTRY,**
**vol. 28, no. 1, janvier 1982, pages 69-74, Easton,**
**Pennsylvania, US; P.C. KAO et al.: "Development and**
**validation of a new radioimmunoassay for parathyrin**
**(PTH)"**

(73) Titulaire: **IRE MEDGENIX S.A., B-6220 Fleurus(BE)**

(72) Inventeur: **Wantier, Henri, 31 Rue d'Italie,**
**B-7370 Elouges(BE)**
Inventeur: **Saussez, Christian, 116 avenue du Haut-Pays,**
**B-7273 Athis(BE)**
Inventeur: **Delacroix, Dominique, 11 avenue des**
**Ramiers, B-1950 Kraainem(BE)**

(74) Mandataire: **Ahner, Francis et al, CABINET**
**REGIMBEAU, 26, avenue Kléber, F-75116 Paris(FR)**

## Description

La présente invention concerne un procédé de dosage de l'hormone parathyroidienne, ou parathormone (PTH) dans un échantillon biologique.

La PTH est une hormone polypeptidique synthétisée par les glandes parathyroidiennes. On la trouve essentiellement dans le sang, soit entière, soit sous la forme de fragments. Ces fragments peuvent être actifs (terminaison $NH_2$) ou inactifs (terminaison COOH).

La PTH est très vite dégradée dans le sang (demivie inférieure ou égale à 20 minutes).

La PTH possède des actions physiologiques variées ; elle intervient notamment dans le métabolisme du calcium et du phosphore et favorise l'absorption intestinale de la vitamine D ; la régulation de la sécrétion de la PTH est liée à la fraction ionisée du calcium plasmatique.

Les dérèglements des glandes parathyroïdes sont de deux ordres : les hypofonctionnements ou hypoparthyroidie et les hyperfonctionnements ou hyperparathyroidies ; ces dernières peuvent être "secondaires" ou réactionelles dans le cas d'un appauvrissement du sang en calcium ; elles peuvent aussi être "primaires", dues notamment à des tumeurs parathyroidiennes, et ayant alors de grandes manifestations osseuses ou viscerales.

Pour détecter tous ces dérèglements, et en particulier pour détecter à temps une tumeur, il est nécessaire de pouvoir évaluer la concentration en parathormone dans un échantillon biologique, plus particulièrement dans le sang, le sérum ou le plasma.

La technique de dosage connue pour être la plus appropriée est une technique immunologique.

Les documents Clinical Chemistry, vol. 28, no 1, janvier 1982, pages 69–74, P.C. KAO etal.; US-A 4 369 138 et US-A 4 508 828 décrivent le dosage de la PTH à l'aide d'immuno-essais.

Il est connu aussi que la performance d'un immuno-essai de la parathormone repose sur sa capacité à discriminer les patients atteints d'hyperparathyroïdie, notamment primaire, et les patients normaux.

Or, pour la plupart des dosages, on note l'apparition d'échantillons "faussement positifs" dans la population normale, rendant la discrimination entre normaux et hyperparathyroïdie primaire à l'aide d'immuno-essais extrêmement hasardeuse.

Cette élévation anormale des taux de parathormone chez des sujets normaux est due à la présence de protéines interférentes dans le plasma ou de protéines vectrices de la parathormone qui joueraient un rôle de réservoir à parathormone.

La présente invention propose un procédé permettant d'éliminer ces interférences. Pour ce faire, l'échantillon biologique dans lequel on doit effectuer le dosage, est préalablement soumis à un extraction des protéines interférentes ou vectrices de la PTH.

Cette extraction est réalisée à l'aide de polyéthylène glycol (PEG), qui sera, de préférence du PEG 6000.

A la suite de ce traitement par le PEG, on effectue selon la présente invention un dosage par une technique immunologique connue, de préférence par une technique radio-immunologique.

L'échantillon biologique utile au procédé de dosage selon la présente invention est, de préférence, un échantillon sanguin, serique ou plasmatique.

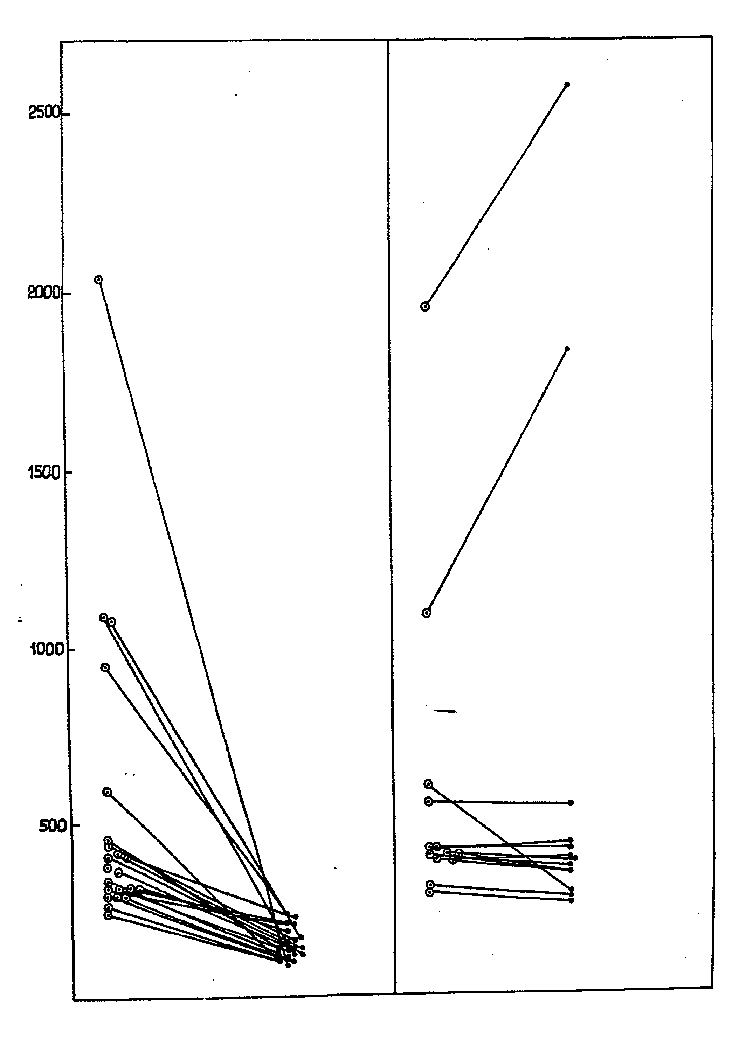

La présente invention sera mieux comprise à l'aide des exemples et de la figure unique qui représente les teneurs en PTH établies :

- ⊙ avant, et
- ■ après,

le traitement de l'échantillon plasmatique par le PEG 6000 chez 22 sujets normaux (colonne de gauche) et chez 13 sujets atteints d'hyperthyroidie primaire (colonne de droite) .

EXEMPLE 1 : SCHEMA DE L'ESSAI

Traitement des échantillons par le PEG 6000

Dans un tube à essai en polystyrène, on ajoute 0,2 ml de PEG 6000 (40 % poids/volume) à 0,4 ml de préparation d'étalonnage ou d'échantillon plasmatique.

Après avoir vortexé le contenu des tubes, ceux-ci sont centrifugés durant 15 minutes à une vitesse de 3000 tours/ minute.

Le surnageant de chaque tube est alors récupéré et employé pour le dosage.

Dosage de la parathormone présente dans le surnageant à l'aide d'un radio-immuno-essai

L'antisérum utilisé a été obtenu chez le lapin par immunisation avec de la parathormone bovine.

La préparation de référence a été réalisée avec le fragment synthétique 44-68 de la parathormone humaine. Du fragment synthétique 44-68 tyrolisé en position 43 a été marqué à l'iode 125.

0,1 ml de surnageant (préparation de référence à différentes doses ou échantillons plasmatiques) et 0,1 ml d'antisérum dilué sont incubés durant 1 nuit à 4°C. On ajoute ensuite 0,1 ml de traceur (±7,4 x$10^2$bq) z. (±0,02 µci). Après une incubation de 4 heures à 4°C, un antisérum dirigé contre les gamma-globulines de lapin et contenant du PEG 6000 (6 % poids/volume) est ajouté à tous les tubes.

Après une incubation de 20 minutes à température ambiante, tous les complexes antigène-anticorps sont précipités. Les tubes sont centrifugés durant 15 minutes à 3000 tours/minute et la radioactivité continue dans le culot est déterminée. Une courbe de référence est construite et les concentrations en parathormone contenues dans les échantillons sont déterminées par interpolation à partir de cette courbe.

EXEMPLE 2 : RESULTATS OBTENUS A L'ISSU DE L'ESSAI SCHEMATISE A L'EXEMPLE 1

22 plasmas de sujets normaux et 13 plasmas de sujets atteints d'hyperparthyroïdie primaire ont été dosés avant et après traitement par le PEG 6000.

Comme montré sur la figure en annexe, 5 des 22 patients normaux dont les taux de parathormone sont supérieurs à 500 picogrammes/ml (pg/ml) présentent une diminution importante en taux de parathormone après traitement au PEG. Pour les 17 autres cas normaux, on note également une légère diminution des taux de PTH après traitement.

Par contre, chez les patients atteints d'hyperparthyroïdie primaire, on observe après traitement au PEG 6000 une augmentation importante des taux de parathormone chez 2 patients dont les valeurs en parathormone sont déjà fort élevées avant traitement. Une diminution du taux de parathormone après traitement est observée chez un patient. Dans les autres cas d'hyperparathyroïdie primaire analysés on n'observe pas de variation significative avant et après traitement par le PEG 6000.

Le tableau ci-dessous résume les valeurs moyennes, extrêmes obtenues dans les différents cas.

| | Sujets normaux (n = 22) | | Sujets hyperparathyroïdiens primaires (n = 13) | |
|---|---|---|---|---|
| | Avant traitement au PEG 6000 | Après traitement au PEG 6000 | Avant traitement au PEG 6000 | Après traitement au PEG |
| Valeur moyenne (ng/ml) | 531 | 153 | 606 | 671 |
| Extrêmes (ng/ml) | 252 – 2043 | 100 – 236 | 308 – 1956 | 279 – 2585 |

Il en résulte donc, après traitement des échantillons par le PEG, et contrairement aux résultats obtenus sans extraction, une parfaite discrimination entre population normale et population de sujet atteints d'hyperparathyroïdie primaire.

**Revendications**

1. Procédé de dosage du type immuno-essai permettant d'évaluer la concentration en PTH dans un échantillon biologique, caractérisé en ce que l'échantillon biologique est, préalablement au dosage proprement dit, soumis à une extraction des protéines interférentes ou vectrices de la parathormone PTH à l'aide de polyéthylène glycol PEG.

2. Procédé selon la revendication 1, caractérisé en ce que le PEG est du PEG 6000.

3. Procédé selon la revendication 1 ou 2, caractérisé en ce que l'échantillon biologique est du sang, du sérum ou du plasma.

4. Procédé selon les revendications 1 à 3, caractérisé en ce que l'immuno-essai est un radio-immunoessai.

**Patentansprüche**

1. Bestimmungsverfahren vom Typ eines Immunoassays, das die Ermittlung der Konzentration an PTH in einer biologischen Probe ermöglicht, dadurch gekennzeichnet, daß aus der biologischen Probe vor der eigentlichen Bestimmung störende oder vektorielle Proteine des Parathormons (PTH) mit Hilfe von Polyethylenglykol (PEG) extrahiert werden.

2. Verfahren nach Anspruch 1, dadurch gekennzeichnet, daß es sich bei dem PEG um PEG 6000 handelt.

3. Verfahren nach Anspruch 1 oder 2, dadurch gekennzeichnet, daß es sich bei der biologischen Probe um Blut, Serum oder Plasma handelt.

4. Verfahren nach Ansprüchen 1 bis 3, dadurch gekennzeichnet, daß es sich bei dem Immunoassay um einen Radioimmunoassay handelt.

**Claims**

1. Method of determination of the immunoassay type enabling the PTH concentration to be measured in a biological sample, wherein, prior to the actual determination, the biological sample is subjected to an extraction of the proteins that interfere or are carriers of parathormone (PTH), by means of polyethylene glycol (PEG).

2. Method as claimed in claim 1, wherein the PEG is PEG 6000.

3. Method as claimed in claim 1, wherein the biological sample is blood, serum or plasma.

4. Method as claimed in claim 1, wherein the immunoassay is a radioimmunoassay.

EP 0 265 327 B1